# EUROPÄISCHE PATENTSCHRIFT

(11) **EP 0 578 998 B1**
(45) Veröffentlichungstag und Bekanntmachung des Hinweises auf die Patenterteilung: **08.10.1997**
(21) Anmeldenummer: 93109923.8
(22) Anmeldetag: 22.06.1993
(51) Int. Cl.: A61F 2/04, A61M 29/00

(54) **In der Körper eines Patienten perkutan implantierbare Endoprothese**
Implantable percutaneous endoprosthesis
Endoprothèse percutanée implantable

(30) Priorität: 08.07.1992 DE 4222380
(43) Veröffentlichungstag der Anmeldung: 19.01.1994
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Heinrich, Dr.-Ing.

(56) Entgegenhaltungen:
- WO-A-90/13332
- WO-A-91/12779
- WO-A-92/06734
- DE-A- 4 030 998
- US-A- 4 879 135
- US-A- 5 059 211

## Beschreibung

Die Erfindung betrifft eine in den Körper eines Patienten, insbesondere in ein Blutgefäß oder in eine andere Körperhöhle, mittels eines Katheters perkutan implantierbare und als länglicher Hohlkörper ausgebildete Endoprothese, die - nach lagerichtiger Plazierung bei der Implantation - von einem kleineren Lumen beim Einführen auf ein der Gebrauchslage entsprechendes größeres Lumen veränderbar und mit einem Hüllmaterial versehen ist, welches mit wenigstens einem eingelagerten, im implantierten Zustand der Prothese nach und nach mit vorzugsweise gleichbleibender Rate an den Patienten abzugebenden Medikament ausgerüstet ist.

Eine solche Endoprothese ist bereits aus der internationalen Anmeldung WO 91/12779 bekannt. Derartige Endoprothesen dienen zum Eröffnen oder Erweitern von Gefäßlumen. Zur Behandlungen von Gefäßerkrankungen bzw. zur Reduzierung der im Zusammenhang mit der Implantation derartiger Endoprothesen bestehenden Gefahr von Stenosen und Gefäßverschlüssen sind die Filamente dieser in Form eines Maschenwerks ausgestalteten Endoprothese von einem Hüllmaterial umschlossen, das mit einer entsprechenden Medikamentation ausgestattet ist. Die eingelagerten Medikamente werden nach der Implantation in Abhängigkeit vom bestehenden Diffusionsdruck gleichmäßig abgegeben.

Nachteilig bei der vorbekannten Endoprothese ist, daß die an die Zwischenräume des Filamentgeflechtes anschließenden Gefäßwandungsabschnitte nur unzureichend oder gar nicht medikamentenbeaufschlagt werden. In diesen Bereichen besteht nach wie vor die Gefahr von Gefäßerkrankungen und/oder -verschlüssen.

Der Erfindung liegt daher die Aufgabe zugrunde, eine Endoprothese zu schaffen, die eine örtlich und zeitlich gleichmäßige Medikamentenversorgung der an die Endoprothesenwandung angrenzenden Gefäßwandungen ermöglicht.

Diese Aufgabe wird dadurch gelöst, daß bei der Endoprothese nach dem Oberbegriff des Patentanspruchs 1 das Hüllmaterial der Endoprothese als eine an der Außen- oder Innenwand der Endoprothese angeordnete geschlossene Umkleidung ausgebildet und bei einer Aufweitung der Endoprothese ohne Rissebildung plastisch verformbar ist.

Dadurch, daß der gesamte Wandungsbereich der Endoprothese mit einem als geschlossene Umkleidung ausgebildeten Hüllmaterial bedeckt ist, ist sichergestellt, daß im gesamten an der Endoprothese anliegenden Gefäßwandungsbereich eine gleichbleibende Medikamentengabe erfolgt. Eine regional unterschiedliche Medikamentenversorgung mit der Gefahr lokaler Gefäßerkrankungen ist somit wirksam ausgeschlossen.

Eine weitere Ausgestaltung der Erfindung sieht vor, daß die Endoprothese eine Umkleidung aufweist, die den Stent im nicht aufgeweiteten Zustand unter Ausbildung von Falten umschließt, wobei sich die Falten beim Aufweiten der Prothese auf das größere Lumen glätten. Diese Ausgestaltung der Erfindung hat den Vorteil, daß die Anforderungen an das Hüllmaterial hinsichtlich der plastischen Verformbarkeit geringer sind, weil zumindest ein Teil der beim Aufweiten der Endoprothese notwendigen Verformung durch die Glättung der Falten ausgeglichen wird.

In vorteilhafter Weise umschließt die von dem Hüllmaterial gebildete Umkleidung auch alle stützenden Teile der Endoprothese vollständig. Hierdurch ist der Stent auch im implantierten Zustand zusätzlich stabilisiert.

Gemäß einer anderen Weiterbildung der Erfindung hat es sich als zweckmäßig erwiesen, wenn das die Umkleidung der Prothese bildende Hüllenmaterial nicht nur eine außen und innen auf der Endoprothese aufliegende Schicht bildet, sondern das umhüllende Material alle stützenden Teile der Endoprothese vollständig einschließt, so daß der Stent im implantierten Zustand zusätzlich stabilisiert ist.

Letzteres kann in einfacher Weise dadurch erreicht werden, wenn gemäß einer abermaligen Weiterbildung die zumindest ein eingelagertes Medikament enthaltende Umkleidung durch Einbringen des die Prothese stützenden Hohlkörpers oder Stents in eine Gußform mit flüssigem und danach erhärtendem dehnbarem Hüllenmaterial aufgebracht wird. Dies hat den Vorteil, daß die Wandanteile der umgossenen Endoprothese eine völlig glatte Oberfläche haben.

Aus der DE-OS 35 03 126 ist zwar schon ein Implantat bekannt, bei dem auf der Oberfläche eines rohrförmigen Trägers oder Stents ein Kollagenüberzug mit einem daran gebundenen Medikament aufgebracht ist, aber dieser Überzug ist nur in begrenztem Maße aufdehnbar, wobei die Medikamentenabgabe mit nichtlinearer Rate erfolgt.

Eine andere vorteilhafte Weiterbildung der Erfindung sieht vor, daß die Umkleidung bei etwa halber Maximalaufdehnung des die Prothese stützenden Hohlkörpers oder Stents auf diesen aufgebracht ist, um auch bei maximaler Aufweitung der Prothese eine durchgängige Beschichtung mittels der Umkleidung zu gewährleisten.

Um eine möglichst große Menge medikamentöser Substanzen unter Beibehaltung der günstigen mechanischen Eigenschaften der Prothese aufzunehmen, kann die Umkleidung der Endoprothese auch als schlauchartige Hohlmembran oder Hohlmanschette ausgebildet sein, die um den Stent herumgelegt und befestigt ist. Zweckmäßigerweise kann dabei die schlauchartige Hohlmembran an der Stentinnen- und/oder Außenseite haftend angelegt und um die Enden des Stents herumgeschlagen sein.

Eine ebenfalls sinnvolle Weiterbildung ist dadurch gekennzeichnet, daß die in der Umkleidung enthaltenen Medikamente in dem Hüllenmaterial gelöst oder in Form von Bläschen aufgenommen sind. Dabei können zur Medikamentenabgabe zusätzlich Löcher in der Innen- und/oder Außenwandung der Umkleidung vorgesehen sein, die sich beim Aufweiten auf das größere Lumen der Prothese so erweitern, daß im maximalen Aufweitungszustand der Prothese zusätzlich Medikamente freisetzbar sind.

Zweckmäßigerweise kann die Anzahl der Löcher in der dem Gefäßlumen zugewandten Wandung der Umkleidung von der der Gefäßinnenwand zugewandten Wandung der Umkleidung verschieden sein, so daß durch dieses Verhältnis der Anteil der zum Gefäßlumen bzw. zur Gefäßwand hin erfolgenden Medikamentenabgabe vorgegeben werden kann.

Auch kann das die Umkleidung bildende Hüllenmaterial mit Vorteil biologisch abbaubar sein, wobei die Abbauprodukte keinen ungünstigen Nebenwirkungen hervorrufen dürfen. Wenn das Hüllenmaterial biologisch abbaubar ist, findet die Medikamentenabgabe nicht durch Hinausdiffundieren aus dem Trägermaterial statt, sondern durch Freisetzung beim Abbau des Trägermaterials, in dem das gelöste Medikament bzw. die dieses enthaltenden Bläschen an die Oberfläche des sich abbauenden Materials gelangen und dadurch in Kontakt mit Körperflüssigkeiten kommen. Die Medikamentenabgabe ist somit vom Abbau des Trägermaterials abhängig und die Abbaubarkeit ist einstellbar.

In vorteilhafter Weise kann die Umkleidung der Endoprothese aus Polymeren oder Polymerverbindungen bestehen, insbesondere aus Poly-D,L-Laktid oder aus Poly-D,L-Laktid-co-trimethylenkarbonat oder aus mit Glutalaldehyd verkettetem Albumin, wobei das Glutalaldehyd nach Vernetzung des Albumins wieder entfernt wird, weil Glutalaldehyd thrombogen wirkt. Die Umkleidung kann aber auch aus Polyacryl oder PolyacrylVerbindungen bestehen.

Es sind zwar schon polymerbeschichtete und mit Medikamenten präparierte Stents bekannt, beispielsweise durch R.C. Oppenheim et al. in Proc. Int. Symp. Contr. Rel. Bioact. Mat. 15 (1988), Seiten 52 - 55, aber bei diesen Stents ist lediglich eine biologisch nicht abbaubare Polymerschicht durch Aufsprühen einer Akryl-Dispersion auf den Stent aufgebracht und es sind keine Mittel zum Aufweiten des Prothesenquerschnitts vorgesehen.

Weiter hat es sich als sinnvoll erwiesen, wenn die Umkleidung aus dem Hüllenmaterial mit wenigstens einem eingelagerten Medikament in solcher Weise durchlässig ist, daß - im implantierten Zustand - durch eine Gefäßwand gebildete Metaboliten in die Blutbahn gelangen und z. B. Sauerstoff oder andere Nährstoffe durch die Umkleidung zur Gefäßwand diffundieren können.

Gemäß einer anderen Weiterbildung kann die Umkleidung zusätzlich mit Poren versehen sein, durch die Substanzen der oben angegebenen Art hindurchdiffundieren können. Zweckmäßigerweise sollten die Poren eine Größe von höchstens 0,5 µ im Durchmesser haben, weil bei einer derartigen Porengröße glatte Muskelzellen nicht von einer Arterienwand durch die Poren zum Arterienlumen gelangen können. Dabei kommt es darauf an, daß alle Teile der Endoprothese und insbesondere Überkreuzungen im Maschenwerk von der Umkleidung überdeckt sind. Bei aus Fadenmaterial durch Stricken oder sonstige Arten der Maschenbildung hergestellten Endoprothesen kommt auch solchen Ausbildungen besondere Bedeutung zu, bei denen lediglich das die Endoprothese bildende Fadenmaterial, bei dem es sich in der Regel um Metallträger handelt, vollständig umhüllt ist, wobei es in einfacher Weise gelingt, möglichst große Maschen zu gewinnen.

In vorteilhafter Weise kann die Umkleidung der Prothese aus mehreren Lagen bestehen und jede Lage mit anderen Medikamenten ausgerüstet sein. Dabei hat sich als zweckmäßig erwiesen, wenn die einzelnen Lagen der Umkleidung aus Materialien verschieden schneller biologischer Abbaubarkeit bestehen. Insbesondere kann die innere Lage der Umkleidung schneller biologisch abbaubar sein als die äußere Lage.

Weiter hat sich als zweckmäßig erwiesen, bei einer mehrlagigen Umkleidung die innere Lage mit antithrombotischen und die äußere Lage mit antiproliferativen und/oder anderen medikamentösen Substanzen auszurüsten. Wenn bei dieser Ausbildung die innere Lage schneller biologisch abbaubar ist als die äußere Lage, wird dadurch der Gefahr einer Thrombose wirksam begegnet, die in den ersten Tagen nach der Implantation besteht. Die antiproliferative Wirkung muß hingegen über einen längeren Zeitraum aufrechterhalten bleiben, der wenigstens sieben Wochen betragen sollte. Dies kann durch die langsamere Abbaubarkeit der äußeren Lage erreicht werden.

Eine andere wichtige Weiterbildung sieht vor, daß die mit antiproliferativen und/oder anderen medikamentösen Substanzen ausgerüstete äußere Lage der Umkleidung aus je einer ringförmigen Umhüllung kurzer Längenerstreckung an den beiden Prothesenenden besteht. Diese Maßnahme trägt der im Tierversuch gewonnenen Erkenntnis Rechnung, daß bei einer Prothese mit einer nicht wasserdurchlässigen oder porösen inneren und äußeren Umkleidung sich relativ schnell nach der Implantation an den Enden Einengungen bilden, und zwar bedingt durch Thrombosen und auch durch Intimaproliferationen. Dabei können die ringförmigen Umhüllungen kurzer Längenerstreckungen an den Prothesenenden auch mit Poren versehen sein. Bei kleinen Poren ist noch immer ein Flüssigkeitsaustausch mit Diffusion gewährleistet. Die Ausrüstung der Enden der Prothese mit Poren wirkt einer Zellwucherung an den Enden entgegen.

Gemäß einer nochmaligen Weiterbildung kann auch die äußere Lage einer mehrlagigen Umkleidung Zytostatika zum Offenhalten einer Tumorstenose enthalten und die innere Lage mit rheologisch günstigen Substanzen ausgerüstet sein, um z. B. bei in den Gallenwegen verwendeten Stents den Gallefluß zu verbessern. Besondere Bedeutung hat diese Maßnahme deshalb, weil es beispielsweise in Gallengangtumorstenosen häufig aufgrund einer sekundären Infektion der Gallengänge zu Klumpenbildungen kommen kann, die am Stent hängenbleiben und so die Passage verstopfen.

Schließlich sieht eine abermalige Weiterbildung der erfindungsgemäßen Endoprothese auch vor, daß diese mit einem sich beim Aufweiten auf ein größeres Lumen stark erweiternden Seitloch ausgerüstet ist, damit durch den umhüllten Stent im Implantationsbereich abgehende Gefäßseitenäste offengehalten werden. Bei Endoprothesen in Form von Drahtgestricken kann das Seitloch darin bestehen, daß ein einzelner Draht einer Masche vor dem Aufweiten durchtrennt ist. Beim Aufweiten eines derartigen Stents entsteht dann ein relativ großes Loch, das als Durchlaß für Blut in einen Seitenast dienen kann. Auch bei zu behandelnden Gallengängen mit Seitästen kann eine Endoprothese mit Seitloch in sinngemäß gleicher Weise benutzt werden. Naturgemäß muß bei der Implantation sichergestellt werden, daß die Prothese mit dem Seitloch lagerichtig zu einem abgehenden Seitenast positioniert wird.

Eine zweckmäßige Ausführungsform kann auch darin bestehen, daß gemäß einer anderen Weiterbildung an der als schlauchartige Hohlmembran ausgebildeten Umkleidung wenigstens ein nach außen führender Zuführschlauch vorgesehen ist, so daß der Hohlraum der Umkleidung kontinuierlich mit Medikamenten versorgt werden kann. Dadurch wird eine Medikamentenabgabe an die Gefäßwand über längere Zeit möglich, wobei im Unterschied zu den sog. "spraying balloons" der Durchfluß im Gefäß erhalten bleibt und die einzubringenden Medikamente bei geringem Druck zugeführt werden können, ohne daß es, wie bei dem vorgenannten Stande der Technik, zu mechanischen Verletzungen der Arterienwand kommen kann.

Nach einer vorteilhaften Weiterbildung kann der Zuführschlauch lösbar an der Umkleidung angeordnet sein, so daß der Zuführschlauch aus der schlauchartigen Hohlmembran nach der Medikamentenabgabe herausgezogen werden kann. Dabei können auch mehrere Zuführschläuche über den Umfang der Umkleidung gleichmäßig verteilt angeordnet sein und jedem Zuführschlauch kann eine Gruppe von Löchern in der Umkleidung zugeordnet sein, um das zu infundierende Medikament über den Umfang der Prothese etwa isotrop einzuleiten und dadurch die Medikamentenabgabe an die umliegende Gefäßinnenwand mit radial gleichmäßigem Druck zu erreichen. Eine andere zweckmäßige Ausführungsform kann darin bestehen, daß die als schlauchartige Hohlmembran ausgebildete Umkleidung, deren Wandung keine Löcher aufweist, zur Aufnahme flüssiger, radioaktiver Stoffe einen nach außen weisenden Zuführschlauch aufweist. Dadurch kann die Gefäßwand temporärer Bestrahlung durch die radioaktiven Substanzen ausgesetzt werden, wobei die Gefahr einer Kontamination des Patientenkörpers vermieden wird.

Schließlich sieht eine Weiterbildung der erfindungsgemäßen Endoprothese vor, daß sich bei einem Geflecht oder Maschenwerk aufweisenden, die Prothese stützenden Hohlkörper deren Lumen durch axialen Zug an der Prothese so verkleinert, daß die Prothese in einen Katheter aufnehmbar und mittels des Katheters aus dem Gefäß entfernbar ist. Dadurch kann die Prothese aus dem Gefäß und damit aus dem Körper des Patienten zurückgezogen werden.

Anhand der beigefügten Zeichnung sollen nachstehend einige Ausführungsformen der erfindungsgemäßen Endoprothese erläutert werden. In schematischen Ansichten zeigen:
- Fig. 1: eine als länglicher Hohlkörper ausgebildete Endoprothese mit einer Umkleidung aus biologisch abbaubarem und mit Medikamenten ausgerüsteten Hüllenmaterial,
- Fig. 2: in gegenüber Fig. 1 vergrößerter Darstellung einen der Schnittlinie II-II in Fig. 1 entsprechenden Längsschnitt durch die Endoprothese,
- Fig. 3: in einer Ausschnittansicht den Aufbau der Endoprothese als aus metallischem Fadenmaterial hergestelltes Gestricke mit locker ineinandergreifenden und Maschen bildenden Schlingen,
- Fig. 4: in einer Ansicht ähnlich Fig. 2 eine Endoprothese mit mehrmaliger Umkleidung und Beschichtung der Prothesenenden mit Medikamenten,
- Fig. 5: in einer gegenüber den Fig. 1 bis 4 verkleinerten Darstellung eine Gefäßprothese mit Seitloch im implantierten Zustand in einer Arterie mit einem von dieser abgehenden Seitast,
- Fig. 6: in einer Ansicht wie in Fig. 5 eine Gefäßprothese mit Seitloch für den Blutabfluß durch einen Hauptgefäßast, während sich der Stent selbst in einem Nebenast forterstreckt,
- Fig. 7: einen Längsschnitt einer in einem Gefäß implantierten Endoprothese mit einer als membranartige Hohlmanschette ausgebildeten Umkleidung, deren Außenwandung mit Löchern zur Medikamentenabgabe versehen ist, und
- Fig. 8: die mit Löchern und Poren versehene Umkleidung in einem Ausschnitt gemäß Fig. 7.

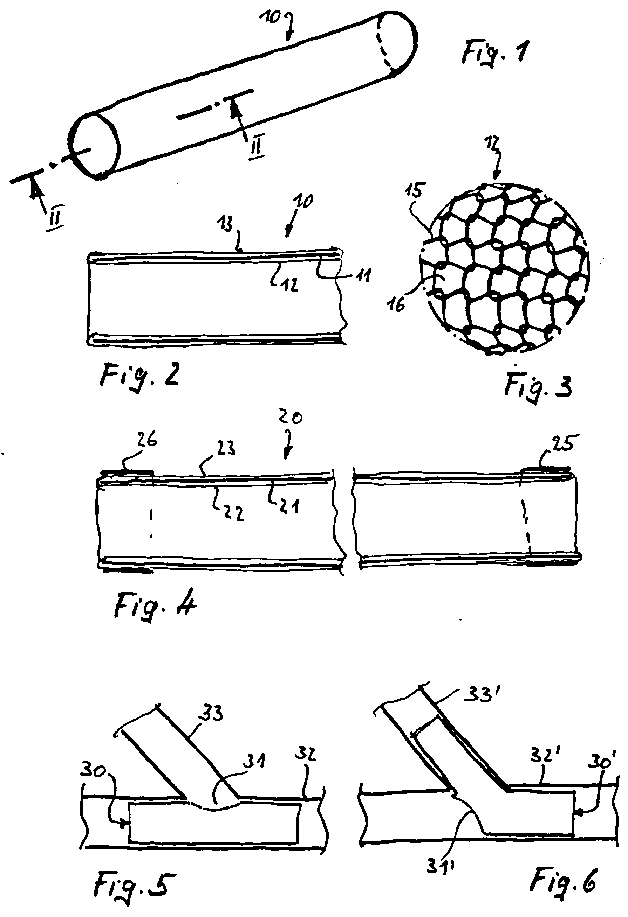

Bei der aus den Fig. 1 und 2 ersichtlichen Endoprothese 10 handelt es sich um einen in seinem Lumen veränderbaren rohrförmigen Hohlkörper, dessen Wandungen 11 von einer inneren und äußeren Umkleidung 12, 13 vollständig eingeschlossen sind. Aufgebracht ist diese Umkleidung auf das eigentliche Prothesenmaterial durch Eintauchen der Prothese in flüssiges Hüllenmaterial, das danach erhärtet ist. Im Hüllenmaterial gelöst sind Medikamente und das Hüllenmaterial ist ohne Bildung schädlicher Abbauprodukte biologisch abbaubar, wodurch nach und nach eine Freisetzung der im Hüllenmaterial im gelösten Zustand eingelagerten Medikamente erfolgt.

Fig. 3 veranschaulicht in einem Wandausschnitt eine als Gestricke aus metallischem Fadenmaterial 15 hergestellte schlauchartige Endoprothese, bei der locker ineinandergreifende Schlingen 16 offene Maschen bilden. Der besondere Vorteil einer derartigen Prothesenausbildung besteht darin, daß diese elastisch nachgiebig ist und nicht nur Gefäßkrümmungen bei der Implantation zu folgen vermag, sondern im implantierten Zustand infolge äußerer Krafteinwirkung auftretende Deformationen werden von einer derartigen Prothese angesichts der ihr eigenen Federrückstellkräfte wieder ausgeglichen.

Bei der aus Fig. 4 ersichtlichen Endoprothese 20 ist die Wandung 21 mit einer inneren und äußeren Umkleidung 22, 23 versehen und an den Prothesenenden sind mehrlagige Umkleidungen 25, 26 aus biologisch abbaubarem Hüllenmaterial angeordnet. Die sich über die gesamte Prothesenlänge erstreckenden Lagen 22, 23 der Umkleidung ist dabei mit antithrombotischen Substanzen ausgerüstet, hingegen die in Form von Endringen nur geringer Axialerstreckung aufgebrachten äußeren Lagen 25, 26 mit antiproliferativen Substanzen, die ein nach längerer Implantationsdauer zu befürchtendes Umwuchern der Prothesenenden wirksam unterbinden, das auf Thrombenbildung oder Intimaproliferation beruht.

Bei einer Prothesengestaltung gemäß Fig. 4 kann es auch angezeigt sein, nur die Umkleidungen 25, 26 der Prothesenenden als Medikamentendepot auszubilden, um die von der Prothese abgegebene Medikamentendosis nicht zu hoch werden zu lassen, damit keine systemische Wirkung erzielt wird.

Bei den erfindungsgemäßen Endoprothesen kann es sich beispielsweise um 4 cm lange sterile Metall-Stents mit einem Innendurchmesser von 4,0 mm handeln, die unter aseptischen Bedingungen in eine Lösung von 4,00 g Poly-D,L-Laktid (inhärente Viskosität = 0,3) + 0,35 g Triacetin + 270 g Aceton getaucht werden. Nach der Trocknung (bei RT 5 Tage, unter vermindertem Druck bei 20 Torr, 16 Tage) und bei 40° C unter vermindertem Druck (4 Tage) hat der Polymerüberzug (24 mg/cm) eine Phasenübergangstemperatur von 25 ⁺ 2° C. Die Polymerlösung kann aber zusätzlich auch 0,40 g Heparin suspendiert enthalten. Der Polymerüberzug ergibt dann 2,0 mg Heparin/cm. Schließlich können, bei einem weiteren Beispiel, die Polymerüberzüge in isotonem Phosphatpuffer pH 7,4 bei 37° C gelagert werden. Bei einem derartigen Versuch setzte die Polymermassenabnahme nach 18 Tagen ein und ergab danach eine Halbwertszeit von 12 Tagen. Die Halbwertszeit der Molmassenabnahme betrug 10 Tage.

Die in Fig. 5 im implantierten Zustand veranschaulichte Endoprothese 30 ist mit einem Seitloch 31 versehen, das beim Aufdehnen der Prothese von einem kleinen Lumen auf ein dem veranschaulichten Implantationszustand entsprechendes größeres Lumen eine starke Vergrößerung erfahren hat und dadurch eine ungehinderte Versorgung des von der die Endoprothese aufnehmenden Arterie 32 abgehenden Seitastes 33 ermöglicht.

Fig. 6 veranschaulicht hingegen eine Endoprothese 30' mit einem Seitloch 31' für den im wesentlichen ungehinderten Blutfluß durch einen Hauptgefäßast 32, während sich der Stent selbst in einem Nebenast 33' forterstreckt. Bei dem Nebenast könnte es sich auch um einen Bypass handeln, in welchem Falle das Seitloch dann koaxial zur Achse des Hauptastes liegt.

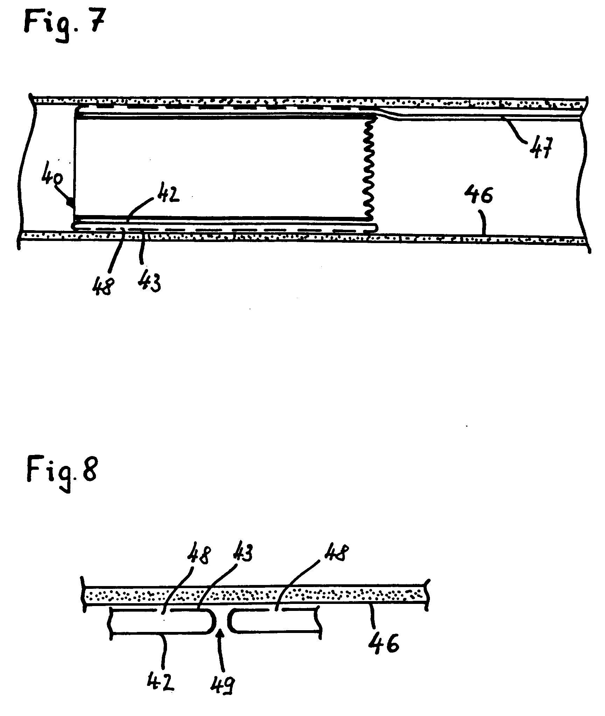

Bei der in Fig. 7 gezeigten Ausführungsform besteht die Endoprothese 40 aus einem Stent und einer diesen umschließenden Umkleidung 42, 43, die als doppelwandige Hohlmanschette ausgebildet ist und deren Außenwandung 43 im implantierten und aufgeweiteten Zustand an einer Gefäßinnenwand 46 und mit ihrer Innenwandung 42 außenseitig am Stent anliegt. Zwischen der Innen- und Außenwandung der Umkleidung befindet sich ein Hohlraum für die Aufnahme von Medikamenten, die über in der Außenwandung 13 angeordnete Löcher 48 an die Gefäßinnenwand 46 abgeben werden können. Auch in der Innenwandung 42 können Löcher vorgesehen sein (nicht gezeigt), und zwar in einer anderen Anzahl als in der Außenwandung 43. Ferner ist ein im Hohlraum zwischen der Innen- und Außenwandung 42, 43 ausmündender Zufuhrschlauch 47 vorgesehen, der sich etwa koaxial zur Axialerstreckung der Prothese 40 entlang der Gefäßinnenwand forterstreckt und eine kontinuierliche Medikamentenzufuhr ermöglicht.

Der Zuführschlauch kann auch lösbar mit der Umkleidung verbunden sein, so daß nach erfolgter Medikamentenabgebe von der Umkleidung eine Abtrennung des Zuführschlauchs vorgenommen werden kann. Dabei kann zum Verschließen der Zuführöffnung an der Umkleidung eine entsprechende Absperreinrichtung vorgesehen sein.

Fig. 8 zeigt einen Abschnitt der membranartigen Umkleidung, bei der zusätzlich zu den für die Medikamentenabgabe bestimmten Löchern 48 in der Außenwandung 43 die Innen- und Außenwandung durchdringende Poren 49 vorgesehen sind. Diese Poren bilden radial verlaufende Verbindungskanäle, um die Diffusion von Metaboliten zwischen Gefäßlumen und Gefäßwand zu ermöglichen.

Bei den den in Fig. 7 und 8 beschriebenen Ausführungsformen der Endoprothese 40 ist eine Medikamentenabgabe an die Gefäßwand 46 über einen längeren Zeitraum möglich, wobei der Durchfluß im Gefäß im wesentlichen unbehindert erhalten bleibt. Die zu infundierenden Medikamente können unter geringem Druck in die schlauchförmige Umkleidung eingeleitet werden, und treten demgemäß auch unter nur geringem Druck durch die sich durch die Wendungen 42, 43 der Umkleidung erstreckenden Löcher aus. Die Gefahr einer mechanischen Verletzung der Gefäßwand 46 ist dabei vernachlässigbar klein.

Weiterhin können auch die zu infundierenden Substanzen in einer geeigneten, definierten Konzentration verabreicht werden, so daß dabei eine Gefäß- oder Zellschädigung weitgehend vermieden wird.

Neben Medikamenten lassen sich zusätzlich Substanzen in die schlauchartige Umkleidung einleiten, durch die der Gefäßwand Nährstoffe zuführbar sind. Insbesondere können Glukose und/oder chemische Puffer wie z.B. Bikarbonat zur Erzielung eines für die Therapierung geeigneten pH-Wertes zugeführt werden. Als Medikamente können einer Arteriosklerose entgegenwirkende oder auch genetische Substanzen verwendet werden, die den Gefäßstoffwechsel regulieren. Zur Verhinderung einer Thrombusbildung an der Innenwand der schlauchförmigen Umkleidung können auch antithrombotische Substanzen verabreicht werden, und zwar vorzugsweise über die Löcher in der Innenwand der Umkleidung.

Die Umkleidung kann bei allen vorstehend beschriebenen Ausführungsbeispielen mit Vorteil beim Aufweiten ohne Rissebildung plastisch verformbar sein. Dies gilt sowohl für Stents, bei denen die Umkleidung aus schlauchlosem ("bulk") Material besteht, wie auch bei den schlauchförmigen Ausführungsformen.

## Patentansprüche

1. In den Körper eines Patienten, insbesondere in ein Blutgefäß oder in eine andere Körperhöhle mittels eines Katheters perkutan implantierbare und als länglicher Hohlkörper ausgebildete Endoprothese (10, 20, 30, 40), die - nach lagerichtiger Plazierung bei der Implantation - von einem kleinen Lumen beim Einführen auf ein der Gebrauchslage entsprechendes größeres Lumen veränderbar und mit einem Hüllmaterial versehen ist, welches mit wenigstens einem eingelagerten, im implantierten Zustand der Prothese nach und nach mit vorzugsweise gleichbleibender Rate an dem Patienten abzugebenden Medikament ausgerüstet ist, dadurch gekennzeichnet,
daß das Hüllmaterial als eine an der Außen- oder Innenwand der Endoprothese (10, 20, 30, 40) angeordnete geschlossene Umkleidung (12, 13; 22, 25, 26; 42, 43) ausgebildet und bei einer Aufweitung ohne Rissebildung plastisch verformbar ist.

2. Endoprothese nach Anspruch 1, dadurch gekennzeichnet, daß das Hüllmaterial als Umkleidung (12, 13; 21, 22, 25, 26; 42, 43) ausgebildet ist, die die Endoprothese (10, 20, 30, 40) unter Ausbildung von Falten umschließt, die sich bei der Aufweitung der Endoprothese (10, 20, 30, 40) glätten.

3. Endoprothese nach Anspruch 1 oder 2, dadurch gekennzeichnet, daß die Umkleidung (12, 13; 22, 23; 42, 43) alle stützenden Teile der Prothese (10, 20, 40) vollständig einschließt.

4. Endoprothese nach Anspruch 1 oder 3, dadurch gekennzeichnet, daß die wenigstens ein eingelagertes Medikament enthaltende Umkleidung (12, 13; 22, 23) durch Einbringen des die Prothese (10, 20) stützenden Hohlkörpers oder Stents in eine Gußform mit flüssigem und danach erhärtendem dehnbarem Hüllenmaterial aufgebracht ist.

5. Endoprothese nach Anspruch 4, dadurch gekennzeichnet, daß die Umkleidung (12, 13; 22, 23) bei etwa halber Maximalaufdehnung des die Prothese (10, 20) stützenden Hohlkörpers oder Stents auf diesen aufgebracht ist.

6. Endoprothese nach einem der Ansprüche 1 bis 3, dadurch gekennzeichnet, daß die Umkleidung (12, 13; 22, 23; 42, 43) aus einer schlauchartigen Hohlmembran besteht, die um die Prothese (10, 20) herumgelegt und befestigt ist.

7. Endoprothese nach Anspruch 6, dadurch gekennzeichnet, daß die schlauchartige Hohlmembran an der Innen- und/oder Außenseite der Prothese (10, 20, 40) haftend angelegt und um die Prothesenenden herumgeschlagen ist.

8. Endoprothese nach einem der Ansprüche 1 bis 7, dadurch gekennzeichnet, daß die in der Umkleidung (12, 13; 22, 23; 42, 43) enthaltenen Medikamente in dem Hüllenmaterial gelöst oder in Form von Bläschen aufgenommen sind.

9. Endoprothese nach Anspruch 6 oder 7, dadurch gekennzeichnet, daß zur Medikamentenabgabe zusätzlich Löcher (48) in der Innen- und/oder Außenwandung der Umkleidung (12, 13; 22, 23; 42, 43) vorgesehen sind, die sich beim Aufweiten auf das größere Lumen so erweitern, daß im maximalen Aufweitungszustand der Umkleidung zusätzlich Medikamente freisetzbar sind.

10. Endoprothese nach Anspruch 9, dadurch gekennzeichnet, daß die Anzahl der Löcher in der dem Gefäßlumen zugewandten Wandung der Umkleidung (12, 13; 22, 23; 42, 43) von der der Gefäßinnenwand zugewandten Wandung der Umkleidung verschieden ist.

11. Endoprothese nach einem der Ansprüche 1 bis 10, dadurch gekennzeichnet, daß das die Umkleidung (12, 13; 22, 23; 42, 43) bildende Hüllenmaterial biologisch abbaubar ist.

12. Endoprothese nach einem der Ansprüche 1 bis 11, dadurch gekennzeichnet, daß die Umkleidung (12, 13; 22, 23; 42, 43) aus Polymeren oder deren Verbindungen, insbesondere aus Poly-D,L-Laktid oder aus Poly-D,L-Laktid-co-trimethylenkarbonat oder aus mit Glutalaldehyd verkettetem Albumin besteht, wobei das Glutalaldehyd nach Vernetzung des Albumins wieder entfernt wird.

13. Endoprothese nach einem der Ansprüche 1 bis 12, dadurch gekennzeichnet, daß die Umkleidung (12, 13, 22, 23, 42, 43) aus Polyacryl oder Polyacryl-Verbindungen besteht.

14. Endoprothese nach einem der Ansprüche 1 bis 13, dadurch gekennzeichnet, daß die Umkleidung (12, 13; 22, 23; 42, 43) in solcher Weise durchlässig ist, daß - im implantierten Zustand - durch eine Gefäßwand gebildete Metaboliten in die Blutbahn gelangen und zum Beispiel Sauerstoff oder andere Nährstoffe vom Blut durch die Umkleidung zur Gefäßwand diffundieren können.

15. Endoprothese nach einem der Ansprüche 1 bis 14, dadurch gekennzeichnet, daß die Umkleidung (12, 13; 22, 23; 42, 43) Poren aufweist, durch die Substanzen hindurchdiffundieren können.

16. Endoprothese nach Anspruch 15, dadurch gekennzeichnet, daß die Poren der Umkleidung (12, 13; 22, 23; 42, 43) eine Größe von höchstens 0,5 Mikrometer im Durchmesser aufweisen.

17. Endoprothese nach einem der Ansprüche 1 bis 16, dadurch gekennzeichnet, daß die Umkleidung (12, 13; 22, 23; 25, 26; 42, 43) aus mehreren Lagen besteht und daß jede Lage mit anderen Medikamenten ausgerüstet ist.

18. Endoprothese nach Anspruch 19, dadurch gekennzeichnet, daß die einzelnen Lagen der Umkleidung (12, 13; 22, 23; 25, 26; 42, 43) aus Materialien verschieden schneller biologischer Abbaubarkeit bestehen.

19. Endoprothese nach Anspruch 18, dadurch gekennzeichnet, daß die innere Lage der Umkleidung (12, 13, 22, 23; 25, 26; 42, 43) schneller biologisch abbaubar ist als die äußere Lage.

20. Endoprothese nach einem der Ansprüche 17 bis 19, dadurch gekennzeichnet, daß die innere Lage der Umkleidung (12, 13, 22, 23; 42, 43) mit antithrombotischen und die äußere Lage mit antiproliferativen und/oder anderen medikamentösen Substanzen ausgerüstet ist.

21. Endoprothese nach Anspruch 20, dadurch gekennzeichnet, daß die mit antiproliferativen und/oder anderen medikamentösen Substanzen ausgerüstete äußere Lage der Umkleidung aus je einer ringförmigen Umhüllung (25, 26) kurzer Längenerstreckung an den beiden Prothesenenden besteht.

22. Endoprothese nach Anspruch 21, dadurch gekennzeichnet, daß die ringförmigen Umhüllungen (25, 26) kurzer Längenerstreckung an den Prothesenenden mit Poren versehen ist.

23. Endoprothese nach einem der Ansprüche 17 bis 22, dadurch gekennzeichnet, daß die äußere Lage der Umkleidung (12, 13; 22, 23; 25, 26; 42, 43) mit Zytostatika zum Offenhalten einer Tumorstenose bzw. als antiproliferative Substanz gegen die Intimahyperplasie und die innere Lage mit rheologisch günstigen Substanzen ausgerüstet ist.

24. Endoprothese nach einem der Ansprüche 1 bis 23, gekennzeichnet durch ein beim Aufweiten auf ein größeres Lumen sich stark erweiterndes Seitloch (31, 31').

25. Endoprothese nach einem der Ansprüche 6 bis 10, dadurch gekennzeichnet, daß an der als schlauchartige Hohlmembran ausgebildeten Umkleidung (42, 43) wenigstens ein nach außen führender Medikamentenzuführschlauch (47) vorgesehen ist.

26. Endoprothese nach Anspruch 25, dadurch gekennzeichnet, daß der Zuführschlauch (47) lösbar an der Umkleidung (42, 43) angeordnet ist.

27. Endoprothese nach Anspruch 25 oder 26, dadurch gekennzeichnet, daß die Zuführschläuche (47) über den Umfang der Umkleidung (42, 43) gleichmäßig angeordnet sind.

28. Endoprothese nach Anspruch 27, dadurch gekennzeichnet, daß jedem Zuführschlauch (47) eine Gruppe von Löchern in der Umkleidung zugeordnet ist.

29. Endoprothese nach einem der Ansprüche 6 bis 8, dadurch gekennzeichnet, daß die als schlauchartige Hohlmembran ausgebildete Umkleidung (42, 43) zur Aufnahme flüssiger, radioaktiver Stoffe einen nach außen führenden Zuführschlauch (47) aufweist.

30. Endoprothese nach einem der Ansprüche 1 bis 29, dadurch gekennzeichnet, daß sich bei einem Geflecht oder Maschenwerk aufweisenden, die Prothese stützenden Hohlkörper deren Lumen durch axialen Zug an der Prothese (10, 20, 40) so verkleinert, daß die Prothese (10, 20, 40) in einen Katheter aufnehmbar und mittels des Katheters aus dem Gefäß entfernbar ist.

## Claims

1. Endoprothesis (10, 20, 30, 40), which is formed as elongate hollow body and percutaneously implantable into the body of a patient, in particular into a blood vessel or into another body cavity, by means of a catheter and which - after positionally correct placing during the implantation - is variable from a small lumen during the introduction to a larger lumen corresponding to the position of use and provided with a sheathing material which is equipped with at least one embedded medicament which in the implanted state of the prothesis is to be delivered gradually at preferably constant rate, characterised thereby, that the sheathing material is formed as a closed coating (12, 13; 22, 25, 26; 42, 43), which is arranged at the outward wall or inward wall of the endoprothesis (10, 20, 30, 40) and which on expansion is plastically deformable without formation of cracks.

2. Endoprothesis according to claim 1, characterised thereby, that the sheathing material is formed as a coating (12, 13; 21, 22, 25, 26; 42, 43), which encloses the endoprothesis (10, 20, 30, 40) whilst forming folds which smooth out on the expansion of the endoprothesis (10, 20, 30, 40).

3. Endoprothesis according to claim 1 or 2, characterised thereby, that the coating (12, 13; 22, 23; 42, 43) completely encloses all supporting parts of the prothesis (10, 20, 30, 40).

4. Endoprothesis according to claim 1 or 3, characterised thereby, that the coating (12, 13; 22, 23) containing at least one embedded medicament is applied by introduction of the hollow body or stent supporting the prothesis (10, 20) into a casting mould with liquid and thereafter hardening expandible sheathing material.

5. Endoprothesis according to claim 4, characterised thereby, that the coating (12, 13; 22, 23) is applied onto the hollow body or stent supporting the prothesis (10, 20) at about half the maximum expansion thereof.

6. Endoprothesis according to one of the claims 1 to 3, characterised thereby, that the coating (12, 13; 22, 23; 42, 43) consists of a hose-like hollow membrane which is laid around the prothesis (10, 20) and fastened.

7. Endoprothesis according to claim 6, characterised thereby, that the hose-like hollow membrane is laid adhesively against the inward side and/or outward side of the prothesis (10, 20, 40) and folded over the ends of the prothesis.

8. Endoprothesis according to one of the claims 1 to 7, characterised thereby, that the medicaments contained in the coating (12, 13; 22, 23; 42, 43) are dissolved in the sheathing material or taken up in the form of small bubbles.

9. Endoprothesis according to claim 6 or 7, characterised thereby, that holes (48) are additionally provided in the inward wall surface and/or the outward wall surface of the coating (12, 13; 22, 23; 42, 43), which holes so enlarge on the expansion to the larger lumen that medicaments are additionally releasable in the state of maximum expansion of the coating.

10. Endoprothesis according to claim 9, characterised thereby, that the number of the holes in that wall of the coating (12, 13; 22, 23; 42, 43), which faces the vessel lumen, differs from that wall of the coating, which faces the inward wall of the vessel.

11. Endoprothesis according to one of the claims 1 to 10, characterised thereby, that the sheathing material forming the coating (12, 13; 22, 23; 42, 43) is biologically decomposable.

12. Endoprothesis according to one of the claims 1 to 11, characterised thereby, that the coating (12, 13; 22, 23; 42, 43) consists of polymers or their compounds, in particular of poly-D, L-lactide or of poly-D, L-lactide-co-cyclopropane carbonate or of albumin linked with glutalaldehyde, wherein the glutalaldehyde is removed again after the cross-linkage of the albumin.

13. Endoprothesis according to one of the claims 1 to 12, characterised thereby, that the coating (12, 13; 22, 23; 42, 43) consists of polyacryl or polyacryl compounds.

14. Endoprothesis according to one of the claims 1 to 13, characterised thereby, that coating (12, 13; 22, 23; 42, 43) is permeable in such a manner that, in the implanted state, metabolites formed by a vessel wall get into the blood stream and, for example, oxygen or other nutrients from the blood, can diffuse through the coating to the vessel wall.

15. Endoprothesis according to one of the claims 1 to 14, characterised thereby, that the coating (12, 13; 22, 23; 42, 43) displays pores, through which substances can diffuse.

16. Endoprothesis according to claim 15, characterised thereby, that the pores of the coating (12, 13; 22, 23; 42, 43) display a size of at most about 0.5 micrometres in diameter.

17. Endoprothesis according to one of the claims 1 to 16, characterised thereby, that the coating (12, 13; 22, 23; 25, 26; 42, 43) consists of several layers and each layer is equipped with other medicaments.

18. Endoprothesis according to claim 19, characterised thereby, that the individual layers of the coating (12, 13; 22, 23; 25, 26; 42, 43) consist of materials of differently rapid biological degradability.

19. Endoprothesis according to claim 18, characterised thereby, that the inner layer of the coating (12, 13; 22, 23; 25, 26; 42, 43) is biologically degradable more rapidly than the outer layer.

20. Endoprothesis according to one of the claims 17 to 19, characterised thereby, that the inner layer of the coating (12, 13; 22, 23; 42, 43) is equipped with antithrombotic substances and the outer layer is equipped with antiproliferative and/or other medicative substances.

21. Endoprothesis according to claim 20, characterised thereby, that the outer layer, which is equipped with antiproliferative and/or other medicative substances, of the coating consists of a respective annular sheathing (25, 26) of short extent in length at each of both the ends of the prothesis.

22. Endoprothesis according to claim 21, characterised thereby, that the annular sheathings (25, 26) of short extent in length at the ends of the prothesis are provided with pores.

23. Endoprothesis according to one of the claims 17 to 22, characterised thereby, that the outer layer of the coating (12, 13; 22, 23; 25, 26; 42, 43) is equipped with cytostatica for keeping a tumour stenosis open or as antiproliferative substance against the intimahyperplasia and the inner layer is equipped with rheologically favourable susbtances.

24. Endoprothesis according to one of the claims 1 to 23, characterised by a side hole (31, 31'), which enlarges greatly during the expansion to a larger lumen.

25. Endoprothesis according to one of the claims 6 to 10, characterised thereby, that at least one medicament feed hose (47) leading to the outside is provided at the coating (42, 43) formed as hose-like hollow membrane.

26. Endoprothesis according to claim 25, characterised thereby, that the feed hose (47) is detachably arranged at the coating (42, 43).

27. Endoprothesis according to claim 25 or 26, characterised thereby, that the feed hoses (47) are arranged uniformly of the circumference of the coating (42, 43).

28. Endoprothesis according to claim 27, characterised thereby, that a group of holes in the coating is associated with each feed hose (47).

29. Endoprothesis according to one of the claims 6 to 8, characterised thereby, that the coating (42, 43) formed as hose-like hollow membrane displays a feed hose (47) leading to the outside for the reception of liquid radio-active substances.

30. Endoprothesis according to one of the claims 1 to 29, characterised thereby, that in the case of a hollow body which displays a mesh or a meshed network and supports the prothesis, the lumen thereof so decreases by axial pull at the prothesis (10, 20, 40) that the prothesis (10, 20, 40) is receivable in a catheter and is removable from the vessel by means of the catheter.

## Revendications

1. Endoprothèse (10, 20, 30, 40), implantable par voie percutanée à l'aide d'un cathéter dans le corps d'un patient, en particulier dans un vaisseau sanguin ou dans une autre cavité corporelle, et conçue comme un objet creux de grande longueur, endoprothèse qui - après mise en place correcte lors de l'implantation - peut passer d'une petite lumière lors de l'introduction à une lumière plus grande correspondant à la position d'utilisation, et qui est pourvue d'un matériau de gainage, lequel est garni d'un médicament incorporé qui, à l'état implanté de la prothèse, est cédé petit à petit au patient, à une vitesse de préférence constante, caractérisée en ce que le matériau de gainage est réalisé sous forme d'un revêtement fermé (12, 13 ; 22, 25, 26 ; 42, 43) disposé contre la paroi extérieure ou la paroi intérieure de l'endoprothèse (10, 20, 30, 40), et peut subir une déformation plastique sans fissuration lors d'un élargissement.

2. Endoprothèse selon la revendication 1, caractérisée en ce que le matériau de gainage est réalisé sous forme d'un revêtement (12, 13 ; 21, 22, 25, 26 ; 42, 43), qui entoure l'endoprothèse (10, 20, 30, 40) avec formation de plis qui se lissent lors de l'élargissement de l'endoprothèse (10, 20, 30, 40).

3. Endoprothèse selon la revendication 1 ou 2, caractérisée en ce que le revêtement (12, 13 ; 22, 23 ; 42, 43) entoure complètement toutes les pièces portantes de la prothèse (10, 20, 40).

4. Endoprothèse selon la revendication 1 ou 3, caractérisée en ce que le revêtement (12, 13 ; 22, 23) qui contient au moins un médicament incorporé, est appliqué par incorporation du corps creux ou du stent qui soutient la prothèse (10, 20) dans un moule de coulée, avec un matériau de gainage liquide, qui ensuite se dilate en durcissant.

5. Endoprothèse selon la revendication 4, caractérisée en ce que le revêtement (12, 13 ; 22, 23), pour un élargissement égal à la moitié de l'élargissement maximal du corps creux ou du stent qui soutient la prothèse (10, 20), est appliqué sur ce dernier.

6. Endoprothèse selon l'une des revendications 1 à 3, caractérisée en ce que le revêtement (12, 13 ; 22, 23 ; 42, 43) est constitué d'une membrane creuse tubulaire, qui est disposée autour de la prothèse (10, 20) et y est fixée.

7. Endoprothèse selon la revendication 6, caractérisée en ce que la membrane creuse tubulaire est disposée d'une manière adhérente sur la face intérieure et/ou sur la face extérieure de la prothèse (10, 20, 40) et est appliquée autour des extrémités de la prothèse.

8. Endoprothèse selon l'une des revendications 1 à 7, caractérisée en ce que les médicaments contenus dans le revêtement (12, 13 ; 22, 23 ; 42, 43) sont dissous dans le matériau de gainage ou y sont logés sous forme de petites bulles.

9. Endoprothèse selon la revendication 6 ou 7, caractérisée en ce que, pour permettre la libération du médicament, on prévoit en plus des perforations (48) dans la paroi intérieure et/ou extérieure du revêtement (12, 13 ; 22, 23 ; 42, 43), perforations qui, lors de l'élargissement à la lumière la plus grande, s'élargissent de façon qu'en outre des médicaments puissent se libérer dans l'état d'élargissement maximal du revêtement.

10. Endoprothèse selon la revendication 9, caractérisée en ce que le nombre des perforations dans la paroi, dirigée vers la lumière du vaisseau, du revêtement (12, 13 ; 22, 23 ; 42, 43) est différent de celui que l'on a dans la paroi du revêtement dirigée vers la paroi vasculaire intérieure.

11. Endoprothèse selon l'une des revendications 1 à 10, caractérisée en ce que le matériau de gainage formant le revêtement (12, 13 ; 22, 23 ; 42, 43) est biodégradable.

12. Endoprothèse selon l'une des revendications 1 à 11, caractérisée en ce que le revêtement (12, 13 ; 22, 23 ; 42, 43) est constitué de polymères ou de leurs composés, en particulier d'un poly-D,L-lactide ou d'un copolymère de D,L-lactide et de carbonate de triméthylène, ou encore d'albumine avec formation de chaînes glutaraldéhyde, où le glutaraldéhyde est de nouveau éliminé après réticulation de l'albumine.

13. Endoprothèse selon l'une des revendications 1 à 12, caractérisée en ce que le revêtement (12, 13 ; 22, 23 ; 42, 43) est constitué d'un polyacrylique ou de composés polyacryliques.

14. Endoprothèse selon l'une des revendications 1 à 13, caractérisée en ce que le revêtement (12, 13 ; 22, 23 ; 42, 43) est perméable en ce sens que les métabolites formés à travers une paroi vasculaire arrivent dans la circulation sanguine, et par exemple de l'oxygène ou d'autres nutriments du sang peuvent, en traversant le revêtement, diffuser vers la paroi vasculaire.

15. Endoprothèse selon l'une des revendications 1 à 14, caractérisée en ce que le revêtement (12, 13 ; 22, 23 ; 42, 43) comporte des pores par lesquels les substances peuvent passer pour subir une diffusion.

16. Endoprothèse selon la revendication 15, caractérisée en ce que les pores du revêtement (12, 13 ; 22, 23 ; 42, 43) ont un diamètre d'au plus 0,5 µm.

17. Endoprothèse selon l'une des revendications 1 à 16, caractérisée en ce que le revêtement (12, 13 ; 22, 23 ; 25, 26 ; 42, 43) est constitué de plusieurs couches, et que chaque couche est garnie d'autres médicaments.

18. Endoprothèse selon la revendication 19, caractérisée en ce que les différentes couches du revêtement (12, 13 ; 22, 23 ; 25, 26 ; 42, 43) sont constituées de matériaux ayant des vitesses de biodégradation différentes.

19. Endoprothèse selon la revendication 18, caractérisée en ce que la couche intérieure du revêtement (12, 13 ; 22, 23 ; 25, 26 ; 42, 43) subit une biodégradation plus vite que la couche extérieure.

20. Endoprothèse selon l'une des revendications 17 à 19, caractérisée en ce que la couche intérieure du revêtement (12, 13 ; 22, 23 ; 42, 43) est garnie de substances anti-thrombotiques, et que la couche extérieure est garnie de substances antiprolifératives et/ou d'autres substances médicamenteuses.

21. Endoprothèse selon la revendication 20, caractérisée en ce que la couche extérieure du revêtement, garnie de substances antiprolifératives et/ou d'autres substances médicamenteuses, est constituée, en chacune des deux extrémités de la prothèse, d'un gainage annulaire (25, 26) ayant une courte étendue longitudinale.

22. Endoprothèse selon la revendication 21, caractérisée en ce que les gainages annulaires (25, 26) ayant une courte étendue longitudinale sont pourvus de pores aux extrémités de la prothèse.

23. Endoprothèse selon l'une des revendications 17 à 22, caractérisée en ce que la position extérieure du revêtement (12, 13 ; 22, 23 ; 25, 26 ; 42, 43) est garnie de cytostatiques pour maintenir ouverte une sténose tumorale, ou servant de substance antiproliférative contre une hyperplasie de l'intima, et la couche intérieure est garnie de substances ayant un effet rhéologique positif.

24. Endoprothèse selon l'une des revendications 1 à 23, caractérisée par un trou latéral (31, 31'), qui s'élargit fortement lors de l'élargissement à une lumière plus grande.

25. Endoprothèse selon l'une des revendications 6 à 10, caractérisée en ce qu'au moins un tuyau souple (47) dirigé vers l'extérieur, destiné à amener les médicaments, est prévu contre le revêtement (42, 43) réalisé sous forme d'une membrane creuse tubulaire.

26. Endoprothèse selon la revendication 25, caractérisée en ce que le tuyau d'amenée (47) est disposé contre le revêtement (42, 43) d'une manière amovible.

27. Endoprothèse selon la revendication 25 ou 26, caractérisée en ce que le tuyau d'amenée (47) est disposé uniformément sur la périphérie du revêtement (42, 43).

28. Endoprothèse selon la revendication 27, caractérisée en ce qu'à chaque tuyau d'amenée (47) est affecté un groupe de perforations dans le revêtement.

29. Endoprothèse selon l'une des revendications 6 à 8, caractérisée en ce que le revêtement (42, 43) réalisé sous forme d'une membrane creuse tubulaire comporte, pour recevoir des substances liquides radioactives, un tuyau d'amenée (47) dirigé vers l'extérieur.

30. Endoprothèse selon l'une des revendications 1 à 29, caractérisée en ce que, pour des corps creux comportant une tresse ou un article maille et soutenant la prothèse, leur lumière subit, sous l'effet d'une traction axiale exercée sur la prothèse (10, 20, 40), une diminution telle que la prothèse (10, 20, 40) peut être reprise dans un cathéter et être éloignée du vaisseau sanguin à l'aide du cathéter.
